# EUROPÄISCHE PATENTSCHRIFT

(11) **EP 0 896 528 B1**
(45) Veröffentlichungstag und Bekanntmachung des Hinweises auf die Patenterteilung: **19.12.2001**
(21) Anmeldenummer: 97920646.3
(22) Anmeldetag: 10.04.1997
(51) Int. Cl.: A61K 9/20, C07C 31/26

(54) **POLYOL-ZUSAMMENSETZUNG**
POLYOL COMPOSITION
COMPOSITION CONSTITUEE DE POLYOLS

(30) Priorität: 22.04.1996 DE 19615418
(43) Veröffentlichungstag der Anmeldung: 17.02.1999
(73) Patentinhaber: MERCK PATENT GmbH, 64293 Darmstadt (DE)
(72) Erfinder: SCHWARZ, Eugen, D-64625 Bensheim (DE); MÖSCHL, Gernot, D-64331 Weiterstadt (DE); MAUL, Karin, D-64289 Darmstadt (DE)
(74) Vertreter: Schüttler, Reinhard, Dr.
(86) Internationale Anmeldenummer: EP9701787
(87) Internationale Veröffentlichungsnummer: WO9739739

(56) Entgegenhaltungen:
- EP-A- 0 380 219
- EP-A- 0 490 768
- EP-A- 0 528 604
- EP-A- 0 645 096
- DE-A- 3 245 170

## Beschreibung

Die Erfindung betrifft eine durch Co-Sprühtrocknung bzw. Co-Wirbelschichtgranulation erhältliche Zusammensetzung, im wesentlichen bestehend aus mindestens zwei Polyolen und ggf. einem Bindemittel, mit einem Gehalt von mindestens einem nicht-hygroskopischen Polyol von mehr als 80 Gew.%, sowie die Verwendung als Tablettierhilfsmittel.

Polyole und Polyolmischungen werden in großem Umfang als nicht-kariogene Zusatzstoffe und Trägerstoffe unter anderem für pharmazeutische Wirkstoffe, Kau- und Lutschtabletten, und andere Produkte der Pharma- und Süßwarenindustrie verwendet. Gewonnen werden Polyole in der Regel durch Hydrierung der ihnen zugrundeliegenden Zucker. In fester Form können sie sowohl durch Kristallisation als auch durch Sprühtrocknung erhalten werden.

Der besondere Vorteil einiger Polyole liegt darin, daß sie auch zum direkten Verpressen ohne weitere Hilfs- und Zusatzstoffe geeignet sind.

Nicht-hygroskopische Polyole sind solche Polyole, die bei einer relativen Luftfeuchtigkeit von 80 % innerhalb von 7 Tagen weniger als 5 % Wasser bei Zimmertemperatur aufnehmen.

Die bekannten Polyole, Mannit, Lactit, Isomalt, Xylit haben eine solche geringe Hygroskopizität, zeigen jedoch schlechtes Tablettierverhalten (geringe Tablettenhärte, Deckeln, hoher Abrieb). Das Erzielen von hohen Tablettenhärten ist grundsätzlich von Vorteil, da Trägerstoffe oftmals nur in geringen Anteilen in pharmazeutischen Formulierungen eingesetzt werden und Wirkstoffe die Tablettenhärten drastisch vermindern können, so daß eine gewünschte Rezeptur nicht tablettierbar ist.

Während Lactit, Isomalt und Xylit in der Komprimatherstellung eher ungebräuchlich sind, wird Mannit in pharmazeutischen Formulierungen durchaus verwendet.

Der Einsatz von Mannit stellt jedoch einen erhöhten Arbeitsaufwand dar, da er in der Regel vor dem Verpressen mit den übrigen Rezepturbestandteilen naßgranuliert werden muß. Im Handel ist auch direkttablettierbarer Mannit, mit dem sich jedoch verglichen mit Sorbit nur unbefriedigende Tablettenhärten erzielen lassen.

Mit Sorbit erhält man, insbesondere bie Sprühtrocknung, sehr gute Tablettenhärten bei entprechend glatter Oberfläche der Komprimate. Die Hygroskopizität von Sorbit ist jedoch deutlich höher als die der anderen Polyole, wodurch seine Verwendbarkeit eingeschränkt ist.

In der DE 32 45 170 wird vorgeschlagen, eine Polyol-Kombination aus Sorbit und 10-15 Gew.% Mannit durch Sprühtrocknung herzustellen. Dadurch soll die Biegefestigkeit der Tabletten erhöht werden. Die Hygroskopizität bleibt jedoch im wesentlichen unbeeinflußt. Es findet sich dort kein Hinweis, daß Polyolkombinationen, die durch Sprühtrocknung hergestellt wurden, mit Mannit als Hauptkomponente verbesserte Eigenschaften, insbesondere hohe Bindekapazität für Wirkstoffe bei geringerer Hygroskopizität erzielt werden können.

In der EP 0 528 604 wird eine durch Co-Schmelzen erhältliche Zusammensetzung aus Sorbit und Xylit beschrieben. Diese führt jedoch zu Tabletten mit vergleichsweise geringer Härte.

Es bestand daher die Aufgabe, eine Polyol-Zusammensetzung zur Verfügung zu stellen, die problemlos herstellbar ist und deren Tablettiereigenschaften, insbesondere hinsichtlich der Tablettenhärte und der Bindekapazität, gegenüber bekannten Polyolen verbessert sind.

Es wurde nun gefunden, daß eine durch Co-Sprühtrocknung erhältliche Polyol-Zusammensetzung enthaltend mindestens 80 Gew.% eines oder mehrerer nicht-hygroskopischer Polyole beim Tablettieren bei gleichem Preßdruck einerseits höhere Tablettenhärten und eine viel glattere Oberfläche ergibt, andererseits gegenüber Sorbit eine deutlich gering Hygroskopizität aufweist.

Gegenstand der Erfindung ist somit eine im wesentlichen aus mindestens zwei Polyolen bestehende, durch Co-Sprühtrocknung erhältliche Zusammensetzung, welche mindestens 80 Gew.% an mindestens einem nicht-hygroskopischen Polyol, insbesondere Mannit, enthält.

Der Begriff Polyol steht für Zuckeralkohole der allgemeinen Formel

CH₂OH-(CHOH)ₙ-CH₂OH,

wobei n für 2 bis 6, vorzugsweise 3 bis 4, steht,
sowie deren dimeren Anhydride, insbesondere C₁₂H₂₄O₁₁.

Insbesondere steht der Begriff Polyole für Hexite wie Sorbit und Mannit, Pentite wie Xylit, möglich sind aber auch C₄-Polyalkohole wie Erythrit oder C₁₂-Polyalkohole wie Lactit. Der Begriff Polyol-Zusammensetzung steht für eine Zusammensetzung aus mehreren Polyolen, die sich in ihrer Zusammensetzung von bei der technischen Herstellung von Mannit anfallenden Zusammensetzungen deutlich unterscheiden, vorzugsweise solche Zusammensetzungen, die mindestens zwei Polyole mit unterschiedlicher Anzahl von C-Atomen enthalten, insbesondere steht der Begriff für eine Zusammensetzung enthaltend Mannit sowie mindestens ein weiteres Hexit, insbesondere Sorbit oder ein Dodecait, insbesondere Lactit.

Bevorzugte Ausführungsformen sind
a) Zusammensetzung erhältlich durch Lösen von mindestens zwei Polyolen in Wasser und Versprühen des erhaltenen wäßrigen Gemisches in einem Luftstrom mit einer Temperatur von 120 bis 300 °C.
b) Zusammensetzung erhältlich durch Lösen von mindestens zwei Polyolen in Wasser und Verwirbelung des erhaltenen Gemisches in einem Luftstrom bei einer Temperatur zwischen 40° und 110 °C.
c) Zusammensetzung, wobei Mannit und Sorbit, Mannit und Lactit oder Mannit, Sorbit bzw. Lactit und weitere Polyole, insbesondere Mannit, Sorbit und Lactit als Polyole eingesetzt werden.
d) Zusammensetzung, wobei das Verhältnis von Mannit zu Sorbit/Lactit in einem Bereich zwischen 80 : 20 und 99 : 1, insbesondere zwischen 90 : 10 und 98 : 2 liegt. In einer besonders bevorzugten Ausführungsform liegt das Verhältinis bei etwa 95 : 5, insbesondere das Verhältnis Mannit : Sorbit:Lactit in einem Bereich von 90 : 1 : 9 bzw. 90 : 9 :1 und 98 : 1 : 1 liegt.
e) Zusammensetzung nach einem der vorangehenden Ansprüche, dadurch gekennzeichnet, daß der Gehalt an Wasser niedriger als 1 Gew.-% liegt.
f) Zusammensetzung, welche 0,05 bis 5 Gew.% an einem Bindemittel enthält.
g) Zusammensetzung, welche Filamente, vorzugsweise nadelförmige Filamente, deren Längen-Breiten-Verhältnis zwischen 15 und 5 zu 1 beträgt, im Kristallgefüge aufweist.

Ein weiterer Gegenstand der Erfindung sind Komprimate, enthaltend eine erfindungsgemäße Zusammensetzung.

Weiterhin Gegenstand der Erfindung ist ein Verfahren zur Herstellung einer im wesentlichen aus mindestens zwei Polyolen und ggf. einem Bindemittel bestehenden Zusammensetzung, beinhaltend die folgenden Schritte:
a) Herstellen einer wäßrigen Lösung von mindestens zwei Polyolen, wobei die Lösung mehr als 80 % eines oder mehrerer nicht-hygroskopischer Polyole, bezogen auf den Gesamtpolyolgehalt enthält,
b1) Versprühen der erhaltenen Lösung in einem Luftstrom mit einer Temperatur zwischen 120 und 300 °C, wobei das Wasser verdampft wird, oder
b2) Verwirbeln der erhaltenen Lösung in einem Luftstrom mit einer Temperatur zwischen 40 und 110 °C, wobei das Wasser verdampft wird.

In einer besonders bevorzugten Ausführungsform besteht die erfindungsgemäße Polyol-Zusammensetzung im wesentlichen aus 85 bis 99 Gew.%, insbesondere 88 bis 98 Gew.%, Mannit, und 5 bis 15 Gew.%, insbesondere 6 bis 12 Gew.%, aus einem oder zwei Polyolen ausgewählt aus Lactit und Sorbit.

Vorzugsweise enthält die erfindungsgemäße Polyol-Zusammensetzung mehr als 90 Gew.%, und weniger als 99 Gew.% Mannit.

Zur Sprühtrocknung wird eine wäßrige Lösung von mindestens zwei Polyolen verwendet. Der Feststoffgehalt wird zuvor vorzugsweise durch Mischen, bei einer Temperatur 80 °C, zweier oder mehrerer Polyol-Lösungen im gewünschten Verhältnis auf etwa 30 bis etwa 75 Gew.%, insbesondere 50 bis 72 Gew.%, eingestellt. Die Versprühung wird durch Zerstäuben mittels Düsen, vorzugsweise mittels eines Zentrifugalzerstäubers, in einen auf eine Temperatur von 120-300 °C, vorzugsweise 140-190 °C erwärmten, trockenen, zentrifugal eingeblasenen Luftstrom durchgeführt. Die Menge der zugeführten Polyollösung und der eingeblasenen Heißluft wird so abgestimmt, daß das Polyol bis auf einen Wassergehalt von etwa 0,3 bis etwa 1 Gew.% getrocknet wird. Auf jeden Fall sollte der Wassergehalt unterhalb 1 Gew.% liegen.

Die Wirbelschichtgranulation wird, wie z. B. in P. Grassmann, F. Widmer "Einführung in die thermische Verfahrenstechnik", Verlag DeGruyter, Berlin 1974, beschrieben, durchgeführt.

Die Polyolagglomerate, die dabei durch Entwässerung der Polyollösungströpfchen erhalten werden, werden bei der Sprühtrocknung auf eine Temperatur von etwa 50 bis etwa 70 °C erwärmt, während sich die eingeblasene Luft auf etwa die gleiche Temperatur abkühlt. Die Polyol-zusammensetzung wird in Behältern gesammelt und ist nach dem Abkühlen direkt zur Herstellung von Tabletten, Komprimaten oder Kaugummi geeignet.

Die so erhaltenen Polyole weisen aufgrund ihrer filamentösen Mikrostruktur eine Bindekapazität für Wirkstoffe auf, die weit höher ist als die von kristallinem Mannit, und der von reinem Sorbit entspricht, ohne jedoch dessen nachteilige Hygroskopizität aufzuweisen.

Die so charakterisierte Polyol-Zusammensetzung besitzt eine Reihe von vorteilhaften Tablettiereigenschaften:

Überraschenderweise kann festgestellt werden, daß mit der erfindungsgemäßen Polyol-Zusammensetzung bei gleicher Preßkraft härtere Tabletten mit deutlich glatterer Oberfläche hergestellt werden können, als mit den bekannten Mannitqualitäten, einschließlich den bekannten DC-Mannitsorten bzw. mechanischen Polyolverreibungen. Die Tablettenhärte bestimmt im wesentlichen das Lutschverhalten. Mit erfindungsgemäß Polyol-Zusammensetzung können optimal glatte, harte Tabletten bereits mit sehr niedrigen Preßkräften hergestellt werden. Tablettiermaschinen, mit denen die erfindungsgemäße Polyol-Zusammensetzung verpreßt wird, können also bei relativ niedrigen Presskräften arbeiten und unterliegen auf diese Weise einem geringeren Verschleiß.

Durch die filamentöse Struktur ist die erfindungsgemäße Polyol-Zusammensetzung in der Lage, auch größere Mengen von Zusatzstoffen, wie z.B. von pharmazeutischen Wirkstoffen, Farbstoffen oder anderen Zusätzen zu binden. Auch bei starker Beladung mit Zusatzstoffen erhält man homogene Mischungen, und die daraus hergestellten Komprimate besitzen ein gleichmäßiges Aussehen.

Aufgrund der besonderen Herstellungsart durch Versprühen einer wäßrigen Lösung ist es möglich, nicht-wasserlösliche und wasserlösliche Zusätze, wie z.B. Zitronensäure, Süßstoffe, insbesondere Acesulfam K, Aspartam®, Saccharin, Cyclamat und Sucralose, Neohesperidin DC, Farbstoffe sowie pharmazeutische Wirkstoffe, vorzugsweise Vitamine, insbesondere Ascorbinsäure und dergleichen, homogen in der Polyol-Zusammensetzung bzw. den daraus hergestellten Tabletten zu verteilen.

Die gegebenenfalls zuzusetzenden Bindemittel sind dem Fachmann geläufig und dienen der Erhöhung der Festigkeit der Zusammensetzung. Als Bindemittel bevorzugt sind Cellulose-Derivate, insbesondere Hydroxypropylmethylcellulose, Carboxymethylcellulose oder Stärke.

Neben der erfindungsgemäßen Polyol-Zusammensetzung enthalten die erfindungsgemäßen Komprimate einen oder mehrere Bestandteile ausgewählt aus:

pharmazeutischen Wirkstoffen und lebensmittelrechtlich zugelassenen Stoffen. Bevorzugte lebensmittelrechtliche zugelassene Stoffe sind natürliche, naturidentische oder künstliche Aroma- oder Geschmacksstoffe, Vitamine, Spurenelemente, Mineralien, Farbstoffe, Gleit-, Trennmittel, Süßstoffe, Stabilisatoren oder Antioxidantien. Der Anteil dieser Bestandteile liegt vorzugsweise zwischen 0,01 und 80 %, insbesondere zwischen 0,1 und 30 %.

Die Herstellung dieser Komprimate erfolgt in an sich bekannter Weise durch Vermischen der Bestandteile in trockener Form und anschließender Tablettierung.

### Herstellungsbeispiele;

### Beispiel 1:

Eine 50%ige wäßrige Lösung, die bezogen auf die Trockenmasse 95 Teile Mannit, 1,5 Teile Hydroxypropylmethylcellulose und 3,5 Teile Sorbit enthält, wird hergestellt.

Diese Polyollösung wird bei etwa 50 °C mittels eines Zentrifugalzerstäubers in den oberen Teil eines zylindrisches Edelstahlturms gesprüht. Gleichzeitig wird auf etwa 160 °C erhitzte Luft sowie Polyolgranulat tangential in die Sprühzone eingeblasen. Der Feststoffstrom wird über eine Kühltrommel abgeführt und dann geteilt: Ein Teil wird in die Sprühzone des Turmes zurückgeführt und der Rest gesiebt, über ein Fließbett nachgetrocknet und anschließend abgefüllt. Das so erhaltene Produkt läßt sich problemlos verpressen und führt zu Tabletten mit sehr glatter Oberfläche.

### Beispiel 2:

Eine 50%ige wäßrige Lösung, die bezogen auf die Trockenmasse 90,5 Teile Mannit und 9,5 Teile Sorbit enthält, wird hergestellt. Das durch Sprühtrocknung analog Beispiel 1 erhaltene Produkt läßt sich problemlos verpressen, wobei Ergebnisse analog den in Beispiel 1 angegebenen erzielt werden.

### Beispiel 3

Eine 50%ige wäßrige Lösung, die bezogen auf die Trockenmasse 95 Teile Mannit und 5 Teile Sorbit enthält, wird hergestellt. Das durch die Sprühtrocknung analog Beispiel 1 erhaltene Produkt läßt sich problemlos verpressen, wobei Ergebnisse analog dem in Beispiel 1 angegeben erzielt werden.

### Beispiel 4

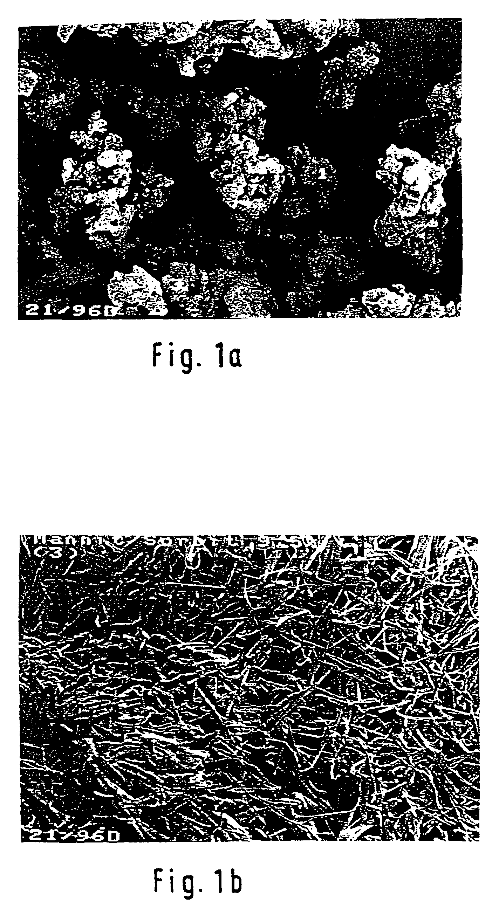

Eine 50%ige wäßrige Lösung, die bezogen auf die Trockenmasse 95 Teile Mannit und 5 Teile Lactit enthält, wird hergestellt. Das durch Sprühtrocknung analog Beispiel 1 erhaltene Produkt läßt sich problemlos verpressen,
wobei Ergebnisse analog dem in Beispiel 1 angegebenen erzielt werden. Die Figuren 1a bis 5b zeigen rasterelektronenmikroskopische Aufnahmen bei einer 50- (Fig. 1a bis 5a) bzw. 5000fachen Vergrößerung (Fig. 1b bis 5b) aufgenommen mit einem Rasterelektronen-Mikroskop Jeol 630 F von verschiedenen Polyolzusammensetzungen.
Fig. 1a zeigt eine 50fache Vergrößerung einer Mannit/Sorbit-Zubereitung erhältlich nach Beispiel 2
Fig. 1b zeigt eine 5000fache Vergrößerung einer Mannit/Sorbit-Zubereitung erhältlich nach Beispiel 2
Fig. 2a zeigt eine 50fache Vergrößerung einer Mannit/Lactit-Zubereitung erhältlich nach Beispiel 4
Fig. 2b zeigt eine 5000fache Vergrößerung einer Mannit-Lactit-Zubereitung erhältlich nach Beispiel 4
Fig. 3a zeigt eine 50fache Vergrößerung eines handelsüblichen DC-Mannits (Pearlitol 300)
Fig. 3b zeigt eine 5000fache Vergrößerung eines handelsüblichen DC-Mannits (Pearlitol 300)
Fig. 4a zeigt eine 50fache Vergrößerung einer mechanischen Verreibung von 90,5 % Mannit und 9,5 % Sorbit
Fig. 4b zeigt eine 5000fache Vergrößerung einer mechanischen Verreibung von 90,5 % Mannit und 9,5 % Sorbit
Fig. 5a zeigt eine 50fache Vergrößerung einer mechanischen Verreibung von 90 % Mannit und 10 % Lactit
Fig. 5b zeigt eine 5000fache Vergrößerung einer mechanischen Verreibung von 90 % Mannit und 10 % Lactit.

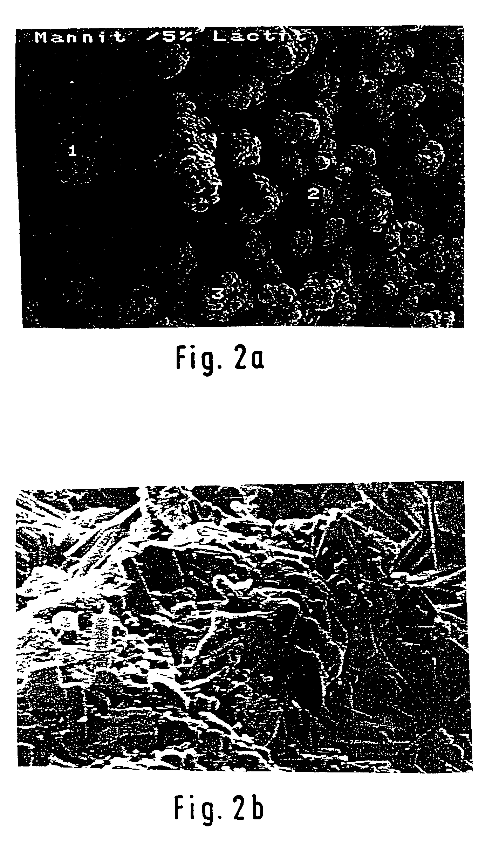

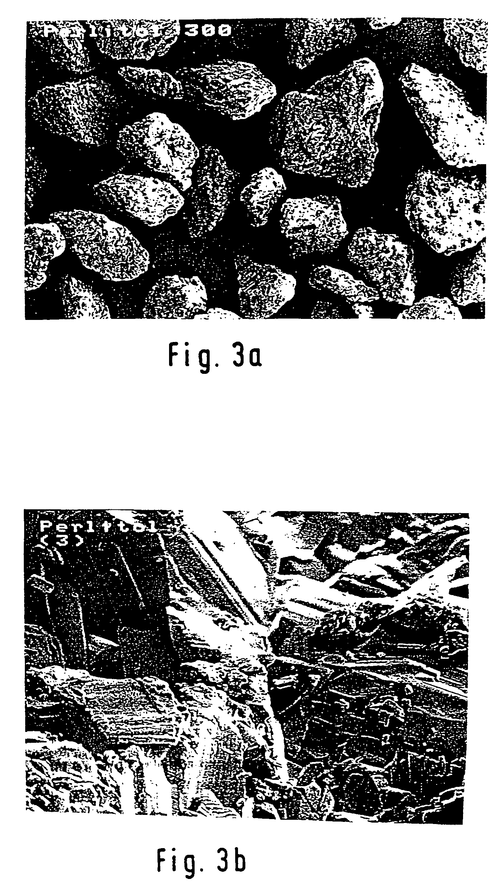

Die Fig. 1b und 2b zeigen deutlich, daß die erfindungsgemäßen Zubereitungen Agglomerate aus extrem feinen nadelförmigen Kristalliten darstellen, wohingegen die Agglomerate von DC-Mannit (Fig. 3b) signifikant unterscheidbar aus größeren Kristalliten bestehen.

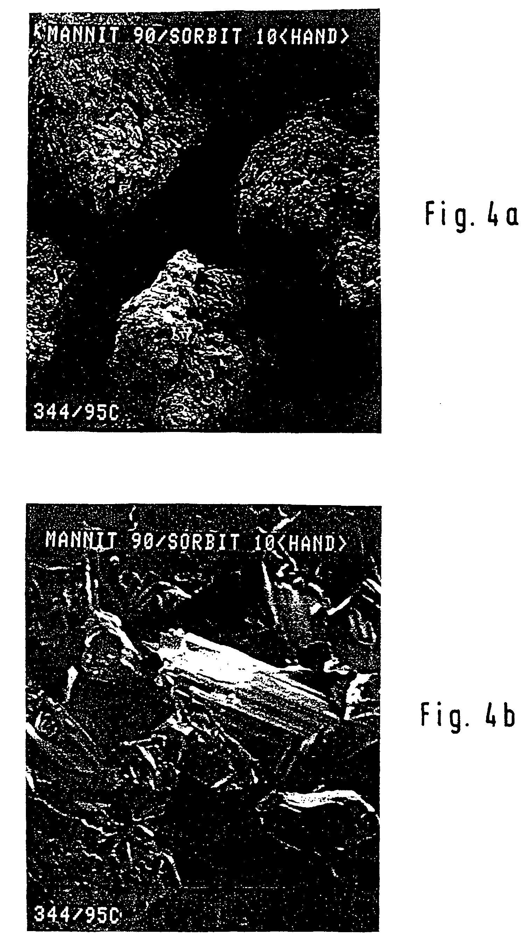

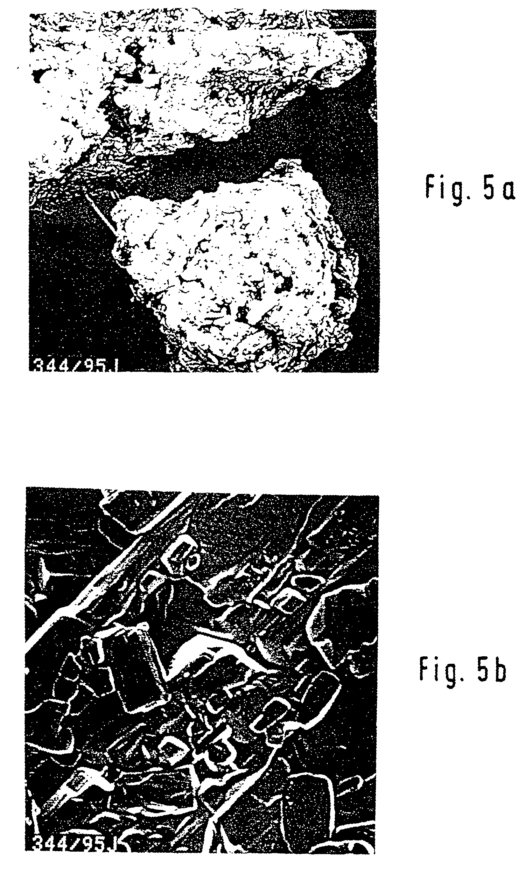

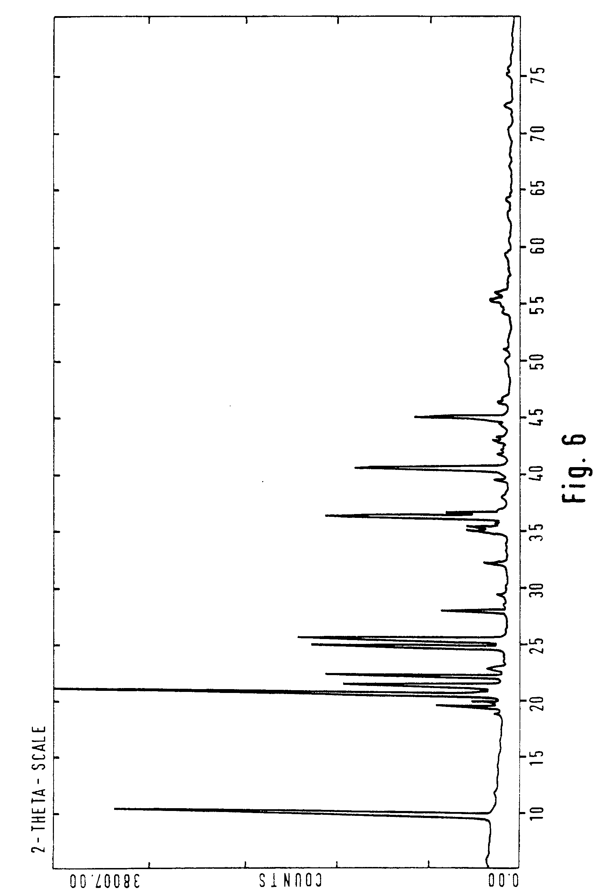

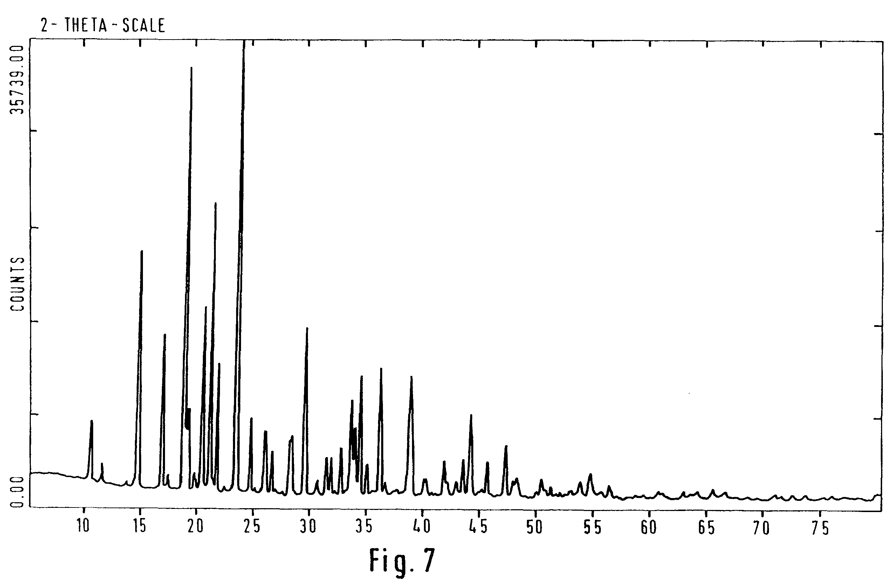

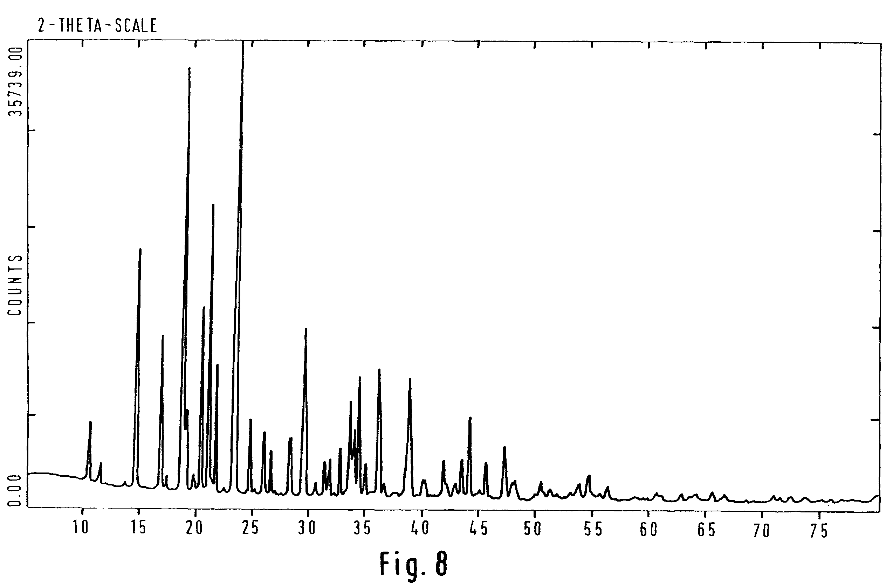

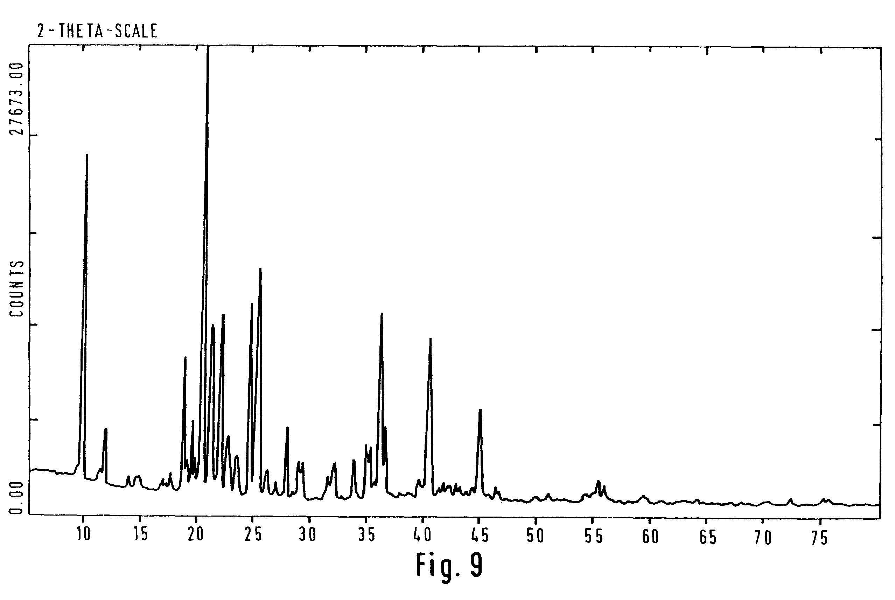

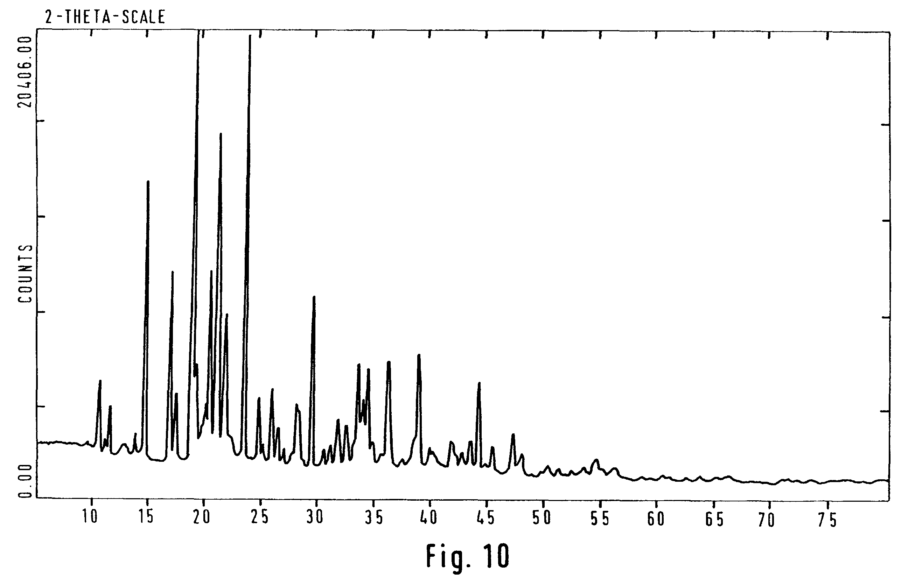

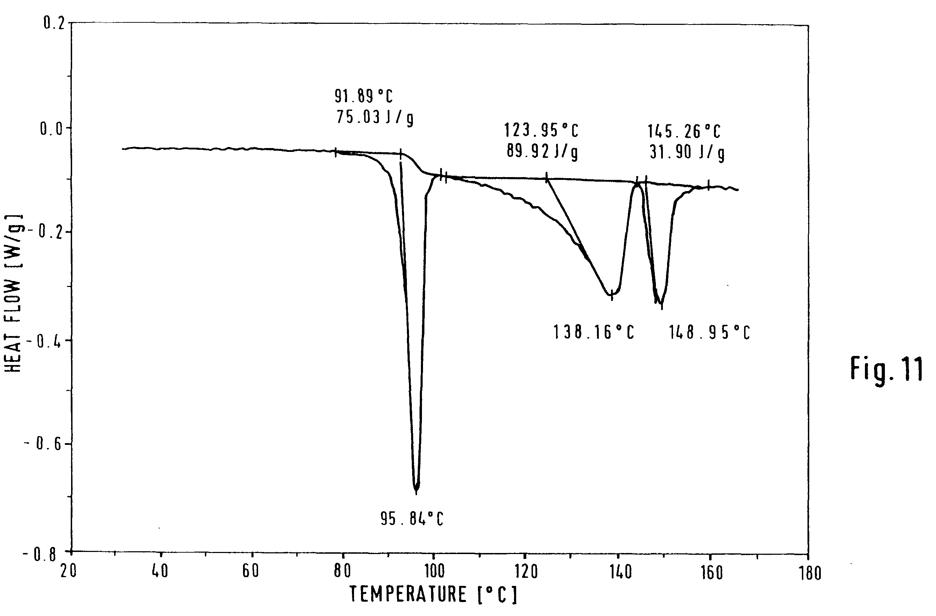

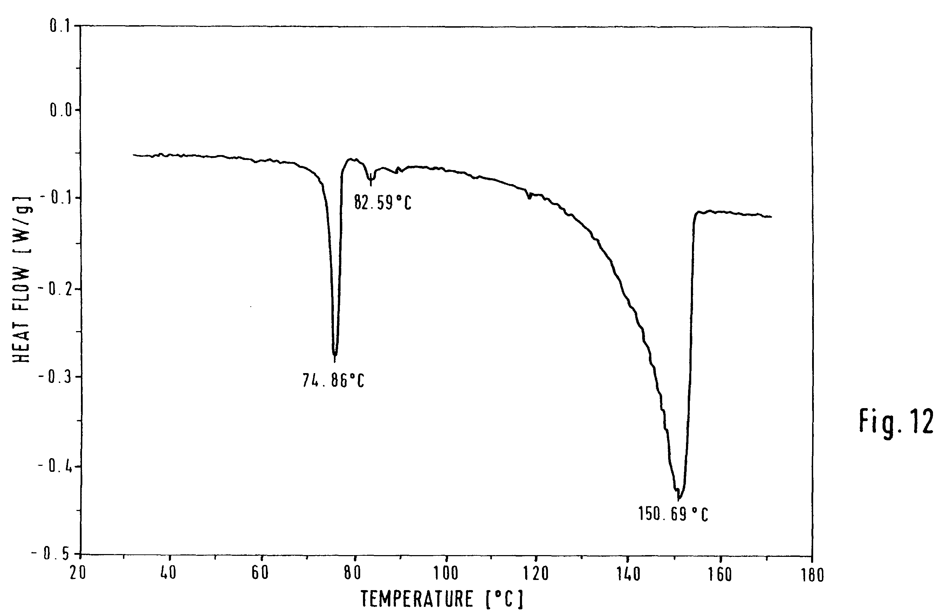

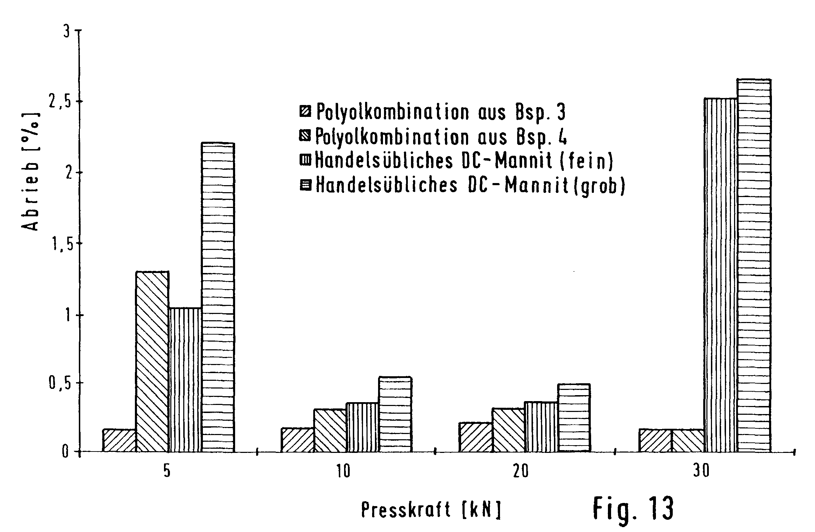

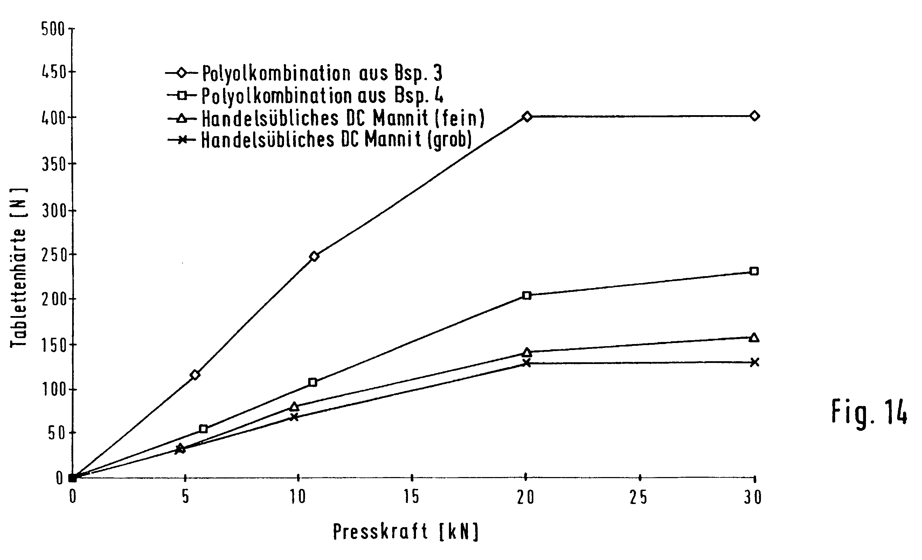

Mechanische Mischungen (Fig. 4b, 5b) können beide Agglomerat-Typen aufweisen.

### Beispiel 5: Lutschtabletten

| | |
|---|---|
| Polyol-Zusammensetzung hergestellt nach Beispiel 2 unter Zusatz von 0,8 Gew.% an Zitronensäure, bezogen auf eingesetztes Polyol | 491,0 Gew.teile |
| Früchtetrockenaroma (verschiedene Geschmacksrichtungen) | 1,5 Gew.teile |
| Magnesiumstearat | 2,5 Gew.teile |

Die Bestandteile werden vermischt und bei einem Preßdruck von 30 kN zu Tabletten von 13 mm Durchmesser und 500 mg Gewicht verpreßt.

### Beispiel 6: Vitamin-C-Tabletten

| | |
|---|---|
| Ascorbinsäure | 105,0 Gew.teile |
| Orangenaroma | 10,0 Gew.teile |
| Polyol-Zusammensetzung hergestellt nach Beispiel 2 | 1377,5 Gew.teile |
| Magnesiumstearat | 7,5 Gew.teile |

Die Bestandteile werden vermischt und bei einem Preßdruck von 11 kN zu Tabletten von 18 mm Durchmesser und 1500 mg Gewicht verpreßt.

### Beispiel 7: Coffeintabletten

| | |
|---|---|
| Polyol-Zusammensetzung nach Beispiel 1 | 462,5 Gew.teile |
| Kaffee-Aroma | 25,0 Gew.teile |
| Coffein | 10,0 Gew.teile |
| Magnesiumstearat | 2,5 Gew.teile |

Die Bestandteile werden vermischt und bei einem Preßdruck von 30 kN zu Tabletten von 13 mm Durchmesser und 500 mg Gewicht verpreßt.

### Beispiel 8: Untersuchung der Tablettiereigenschaften

Es werden mit verschiedenen Polyolen Tabletten hergestellt:

| | | | |
|---|---|---|---|
| Tablettendurchmesser | 11 mm | Tablettengewicht | 450 mg |
| Tablettenhöhe | 3,7 bis 3,9 mm | Preßdruck | 15 kN |

### Eingesetztes Polyol

- A:: Sprühgetrocknetes Mannit : Sorbit = 90,5 : 9,5 aus Beispiel 2
- B:: Sprühgetrocknetes Mannit : Sorbit = 95 : 5 aus Beispiel 3
- C:: Sprühgetrocknetes Mannit : Lactit = 95 : 5 aus Beispiel 4
- D:: Handelsübliches DC-Mannit
- E:: Kristallisiertes Mannit
- F:: Mechanische Verreibung von Mannit mit Sorbit im Verhältnis 95:5
- G:: Mechanische Verreibung von Mannit mit Lactit im Verhältnis 95 : 5
- H:: Sorbit, Instant Pharma, erhältlich von Merck KGaA, Darmstadt

Die Tablettiereigenschaften dieser Produkte können Tabelle I entnommen werden.

**Tabelle I**

| Polyol | A | B | C | D | E | F | G | H |
|---|---|---|---|---|---|---|---|---|
| Tablettenhärte (kN) | 281 | 229 | 195 | 85 | 72 | 85 | 80 | 215 |
| Abrieb (%) | 0,14 | 0,2 | 0,16 | 0,3 | 5,9 | 1,2 | 2,0 | 0,18 |

Die erfindungsgemäßen Tabletten weisen aufgrund ihrer Härte und ihres geringen Abriebs ein angenehmeres Lutschverhalten als die Vergleichsproben (Mannit) auf.

### Beispiel 9

Untersuchung der Hygroskopizität der Polyole.

Die Polyole B, C, D, E, F, H aus Beispiel 8 werden 7 Tage bei einer Luftfeuchte von 76 % gelagert.

Die Wasseraufnahme der Produkte kann Tabelle II entnommen werden

**Tabelle II**

| Polyol | B | C | D | E | F | H |
|---|---|---|---|---|---|---|
| Wasseraufnahme (%) | 1,8 | 0,44 | 0,4 | 0,05 | 1,9 | 5,3 |

Die erfindungsgemäßen hergestellten Polyolgemische zeigen gegenüber handelsüblichem DC-Mannit (D) eine geringfügig höhere Hygroskopizität, jedoch ist diese gegenüber Sorbit (H) deutlich reduziert.

### Beispiel 10

Untersuchung der Beladungskapazität (geordnete stabile Mischungen) der Polyole.

Die Polyole B und D werden über ein Sieb mit einer Porengröße von 200 µm gesiebt und mit 3 Gew.% eines pharmazeutischen Wirkstoffs mit einer Korngröße von weniger als 40 um gemischt.

Das so erhaltene Gemisch wird über einen Luftstrahlsieb von 100 µm kurz abgeblasen. Der im Gemisch zurückbleibende Wirkstoff wird colorimetrisch bestimmt.

Die so bestimmte Beladungskapazität des Trägermaterials (Wiederfindungsrate in %) kann Tabelle III entnommen werden.

| Polyol | B | D | H |
|---|---|---|---|
| Kapazität (bei 3 %) | 83 | 72 | 83 |

Die Beladungskapazität ist deutlich verbessert gegenüber DC-Mannit.

### Beispiel 11

Vergleich des Tablettierverhaltens von Polyol-Zusammensetzungen und handelsüblichem DC-Mannit

Das Prüfmaterial entspricht der gemäß Beispiel 3 hergestellten Zusammensetzung (95 Teile Mannit, 5 Teile Sorbit), welche mit 1% Magnesium-Stearat bezogen auf das Gesamtgewicht vermischt wird und zu Tabletten gepresst wird. Im Vergleich dazu werden gleiche Mengenverhältnisse miteinander verrieben. Weiterhin werden entsprechende Zusammensetzungen untersucht, welche durch Coversprühen von 60 Teilen Mannit mit 40 Teilen Sorbit hergestellt worden sind bzw. durch Verreiben entsprechender Mengenverhältnisse Mannit und Sorbit miteinander, und anschließend vermischt worden sind mit 1 % Magnesium-Stearat bezogen auf die Gesamtmasse.

| | |
|---|---|
| Tablettenpresse | Korsch EK 0 |
| Tablettendurchmesser | 11 mm |
| Presswerkzeuge | flach, facettiert mit Bruchkerbe |
| Tablettengewicht | 500 mg |
| Bruchfestigkeitstester | Erweka TBH 28 (umgerechnet auf Schleuniger) |
| Abriebbestimmung | Erweka Friabilator TA |

Die hergestellten Tabletten wurden Einer Röntgen-Struktur-Analyse und einer DSC-Analyse unterzogen.

Die Röntgenbeugungsanalyse wurde mit einem Pulverdiffraktometer D5000 der Fa. Siemens untersucht.

### Probenvorbereitung:

Ca. 0,5 g der Probe werden in einem Achatmörser leicht gemörsert, auf eine Mylar-Folie aufgebracht und mit einer zweiten Mylar-Folie zugedeckt. Die Mylar-Folie wurde auf einen für das Diffraktometer geeigneten Probenhalter fixiert.

### Meßbedingungen:

Transmissionsbetrieb, Generatorleistung 40 kV/30 mA, Cu-Kα1-Strahlung (Primärmonochromator), ortsempfindlicher Detektor (3,3 kV), Meßbereich: 5° - 80° (2θ); Schrittzeit: 24 s; Schrittgröße: 0,05°.

### Durchführung:

Mit der Messung wird sofort nach der Probenahme begonnen. Das erstellte Röntgendiffraktogramm wird mit den Referenzdiffraktogrammen verglichen.

Die DSC-Analyse (Differential Scanning Calorimetry) wurde mit einer Zelle (System 2100) mit zentralem Computer, Module Interface, DSC-Cellbase und DSC-Zelle der Fa. TA Instruments (früher Du Pont) durchgeführt.

| Meßbedingungen: | |
|---|---|
| Probengefäß | Standard-Pfännchen offen |
| Atmosphäre | 0,15 l/min N₂ |
| Temperaturkalibrierung | o-Terphenyl (T = 55,1 °C) Anissäure (T = 183,2°C) |
| Aufheizrate | 2 °C/min |
| Einsatztemperatur der Probengefäßes | Raumtemperatur |

Die DSC-Messung wird von Raumtemperatur bis 180 °C durchgeführt.
Die DSC-Kurve wird zwischen 50 und 175 °C mit dem Programm "General 4.1" ausgewertet und die Aufheizrate in °C/min mitgeteilt.

Die Darstellungen auf den Seiten 6/14 bis 14/14 haben jeweils folgendes zum Gegenstand:
Fig. 6
   Mannit: Sorbit 95 : 5, verrieben
   Röntgenstrukturanalyse : CT: 24,0s, SS: 0,050 dg, WL: 1,5406
Fig. 7
   Mannit : Sorbit 95 : 5, co-versprüht
   Röntgen-Struktur-Analyse: CT: 24,0 s, SS: 0,050 dg, WL: 15406 A
Fig.:8
   Mannit: Sorbit 95 : 5, co-versprüht
   Röntgen-Struktur-Analyse: CT: 24,0 s, SS: 0,050 dg, WL: 15406 A
Fig.:9
   Mannit: Sorbit 60 : 40, verrieben
   Röntgen-Struktur-Analyse: CT: 24,0 s, SS: 0,050 dg, WL: 15406 A
Fig.:10
   Mannit: Sorbit 60 : 40, co-versprüht
   Röntgen-Struktur-Analyse: CT: 24,0 s, SS: 0,050 dg, WL: 15406 A
Fig.:11
   Mannit: Sorbit 60 : 40, verrieben
   DSC-Analyse
Fig.:12
   Mannit: Sorbit 60 : 40, co-versprüht
   DSC-Analyse
Fig.:13
   Vergleich des Tablettierverhaltens von Polyolkombinationen und handelsüblichem DC-Mannit-Abrieb
Fig.:14
   Vergleich des Tablettierverhaltens von Polyolkombinationen und handelsüblichem DC-Mannit: Tablettenhärten

## Patentansprüche

1. Zusammensetzung im wesentlichen bestehend aus mindestens zwei Polyolen und ggf. einem Bindemittel, mit einem Gehalt von mindestens einem nicht-hygroskopischen Polyol von mehr als 80 Gew.% erhältlich durch Co-Sprühtrocknung bzw. Co-Wirbelschichtgranulation.

2. Zusammensetzung nach Anspruch 1, **dadurch gekennzeichnet, daß** das nicht-hygroskopische Polyol ausgewählt ist aus der Gruppe bestehend aus Mannit, Lactit, Isomalt, Xylit und Erythrit.

3. Zusammensetzung nach Anspruch 1 oder 2, erhältlich durch Lösen von mindestens zwei Polyolen in Wasser und Versprühen des erhaltenen wäßrigen Gemisches in einem Luftstrom mit einer Temperatur von 120 bis 300 °C.

4. Zusammensetzung nach Ansprüchen 1 bis 3, erhältlich durch Lösen von mindestens zwei Polyolen in Wasser und Verwirbelung des erhaltenen Gemisches in einem Luftstrom bei einer Temperatur zwischen 40° und 150 °C.

5. Zusammensetzung nach einem der Ansprüche 1 bis 3, **dadurch gekennzeichnet, daß** Mannit und Sorbit, Mannit und Lactit oder Mannit, Sorbit bzw. Lactit und weitere Polyole, insbesondere Mannit, Sorbit und Lactit als Polyole eingesetzt werden.

6. Zusammensetzung nach Anspruch 5, **dadurch gekennzeichnet, daß** das Verhältnis von Mannit zu Sorbit/Lactit in einem Bereich zwischen 80 : 20 bis 99 : 1, insbesondere zwischen 90 : 10 bis 98 : 2 liegt.

7. Zusammensetzung nach Anspruch 5, **dadurch gekennzeichnet, daß** das Verhältnis Mannit : Sorbit : Lactit in einem Bereich zwischen 90 : 1 : 9 bzw. 90 : 9 : 1 bis 98 : 1 : 1 liegt.

8. Zusammensetzung nach einem der vorangehenden Ansprüche, **dadurch gekennzeichnet, daß** der Gehalt an Wasser niedriger als 1 Gew.-% liegt.

9. Zusammensetzung nach einem der vorangehenden Ansprüche, **dadurch gekennzeichnet, daß** sie 0,05 bis 5 Gew.% an Bindemittel enthält.

10. Zusammensetzung nach einem der vorangehenden Ansprüche, **dadurch gekennzeichnet, daß** sie Filamente in Kristallgefüge aufweist.

11. Tabletten bzw. Komprimate, enthaltend eine Zusammensetzung nach einem der Ansprüche 1 bis 10.

12. Tabletten nach Anspruch 11 enthaltend mindestens einen pharmazeutischen Wirkstoff.

13. Verfahren zur Herstellung einer im wesentlichen aus mindestens zwei Polyolen und ggf. mindestens einem Bindemittel bestehenden Zusammensetzung, beinhaltend die folgenden Schritte:
a) Herstellen einer wäßrigen Lösung von mindestens zwei Polyolen, wobei die Lösung mehr als 80 % eines oder mehrerer nicht-hygroskopischer Polyole bezogen auf den Gesamtpolyolgehalt enthält,
b1) Versprühen der erhaltenen Lösung in einem Luftstrom mit einer Temperatur zwischen 120 und 300 °C, wobei das Wasser verdampft wird, oder
b2) Verwirbeln der erhaltenen Lösung in einem Luftstrom mit einer Temperatur zwischen 40 und 150 °C, wobei das Wasser verdampft wird.

## Claims

1. Composition essentially consisting of at least two polyols and, if appropriate, a binder, having a content of at least one non-hygroscopic polyol of more than 80% by weight obtainable by co-spray-drying or co-fluidized-bed granulation.

2. Composition according to Claim 1, **characterized in that** the non-hygroscopic polyol is selected from the group consisting of mannitol, lactitol, isomaltol, xylitol and erythritol.

3. Composition according to Claim 1 or 2, obtainable by dissolving at least two polyols in water and spraying the resulting aqueous mixture in an air stream at a temperature from 120 to 300°C.

4. Composition according to Claims 1 to 3, obtainable by dissolving at least two polyols in water and vortexing the resulting mixture in an air stream at a temperature between 40 and 150°C.

5. Composition according to one of Claims 1 to 3, **characterized in that** mannitol and sorbitol, mannitol and lactitol or mannitol, sorbitol and lactitol and other polyols, in particular mannitol, sorbitol and lactitol are used as polyols.

6. Composition according to Claim 5, **characterized in that** the ratio of mannitol to sorbitol/lactitol is in a range between 80:20 and 99:1, in particular between 90:10 and 98:2.

7. Composition according to Claim 5, **characterized in that** the ratio mannitol:sorbitol:lactitol is in a range between 90:1:9 or 90:9:1 and 98:1:1.

8. Composition according to one of the preceding claims, **characterized in that** the water content is less than 1% by weight.

9. Composition according to one of the preceding claims, **characterized in that** it comprises from 0.05 to 5% by weight of binder.

10. Composition according to one of the preceding claims, **characterized in that** it has filaments in a crystal structure.

11. Tablets or compressed articles, comprising a composition according to one of Claims 1 to 10.

12. Tablets according to Claim 11 comprising at least one pharmaceutical active compound.

13. Process for preparing a composition essentially consisting of at least two polyols and, if appropriate, at least one binder, comprising the following steps:
a) preparing an aqueous solution of at least two polyols, the solution comprising more than 80% of one or more non-hygroscopic polyols based on the total polyol content,
b1) spraying the resulting solution in an air stream at a temperature between 120 and 300°C, the water being evaporated, or
b2) vortexing the resulting solution in an air stream at a temperature between 40 and 150°C, the water being evaporated.

## Revendications

1. Composition se composant pour l'essentiel d'au moins deux polyols et, le cas échéant, d'un liant, présentant une teneur d'au moins un polyol non hygroscopique de plus de 80% en poids, obtenu par traitement thermique par atomisation de Co ou par granulation en lit fluidisé de Co.

2. Composition selon la revendication 1, **caractérisée en ce que** le polyol non hygroscopique est sélectionné parmi le groupe se composant de mannitol, lactitol, isomalt, xylitol et erythritol.

3. Composition selon la revendication 1 ou 2, obtenue par la dissolution d'au moins deux polyols dans de l'eau et la pulvérisation du mélange aqueux obtenu dans un courant d'air à une température allant de 120 à 300°C.

4. Composition selon l'une quelconque des revendications 1 à 3, obtenue par la dissolution d'au moins deux polyols dans de l'eau et le tourbillonnement du mélange obtenu dans un courant d'air à une température comprise entre 40° et 150°C.

5. Composition selon l'une quelconque des revendications 1 à 3, **caractérisée en ce que** l'on introduit du mannitol et du sorbitol, du mannitol et du lactitol ou du mannitol, du sorbitol et du lactitol ainsi que d'autres polyols, notamment du mannitol, du sorbitol et du lactitol en tant que polyols.

6. Composition selon la revendication 5, **caractérisée en ce que** le rapport mannitol au sorbitol/lactitol se trouve dans une plage comprise entre 80:20 et 99:1, notamment entre 90:10 et 98:2.

7. Composition selon la revendication 5, **caractérisée en ce que** le rapport mannitol:sorbitol:lactitol se trouve dans une plage comprise entre 90:1:9 ou 90:9:1 et 98:1:1.

8. Composition selon l'une quelconque des revendications précédentes, **caractérisée en ce que** la teneur en eau est inférieure à 1% en poids.

9. Composition selon l'une quelconque des revendications précédentes, **caractérisée en ce qu'**elle comprend entre 0,05 et 5% en poids de liant.

10. Composition selon l'une quelconque des revendications précédentes, **caractérisée en ce qu'**elle présente des filaments de structure cristalline.

11. Tablettes ou comprimés comprenant une composition selon l'une quelconque des revendications 1 à 10.

12. Tablettes selon la revendication 11, comprenant au moins un agent pharmaceutique.

13. Procédé destiné à la fabrication d'une composition se composant pour l'essentiel d'au moins deux polyols et, le cas échéant, d'au moins un liant, comprenant les étapes suivantes :
a) fabrication d'une solution aqueuse comprenant au moins deux polyols, moyennant quoi la solution contient plus de 80% d'un ou plusieurs polyol(s) non hygroscopique(s) par rapport à la teneur totale en polyols,
b1) pulvérisation de la solution obtenue dans un courant d'air à une température comprise entre 120 et 300°C, moyennant quoi l'eau est évaporée, ou
b2) tourbillonnement de l'eau obtenue dans un courant d'air à une température comprise entre 40 et 150°C, moyennant quoi l'eau est évaporée.
